# EUROPEAN PATENT APPLICATION

(11) **EP 1 614 436 A1**
(43) Date of publication of application: **11.01.2006**
(21) Application number: 03816652.6
(22) Date of filing: 10.11.2003
(51) Int. Cl.: A61L 27/38, A61K 35/12

(54) **CELLULAR PREPARATION**

(30) Priority: 16.04.2003 JP 2003112103
(71) Applicant: Inoue, Kazutomo, Kyoto-shi, Kyoto 606-8163 (JP); Creative Co., Ltd., Kyoto-shi, Kyoto 600-8095 (JP)
(72) Inventor: INOUE, Kazutomo, Sakyo-ku Kyoto-shi, Kyoto 606-8163 (JP); GU, Yuanjun, Shanghai 201101 (CN); SUMI, Shoichiro, Kyoto 614-8352 (JP)
(74) Representative: Teipel, Stephan
(86) International application number: PCT/JP2003/014271
(87) International publication number: WO 2004/091679

(57) **Abstract**

The present invention provides a cellular preparation wherein cells capable of secreting biologically active factors such as hormones or proteins useful for patients can be retained in polyvinyl alcohol stably for a prolonged period of time and a production method of the cellular preparation, and a method for prevention and treatment of endocrine/metabolic disease, hemophilia, bone disease or cancer by administration of the cellular preparation to patients.

## Description

### TECHNICAL FIELD

The present invention relates to a cellular preparation useful for pharmaceutical compositions for human beings or animals. More particularly, the present invention relates to a cellular preparation wherein cells capable of producing/secreting biologically active factors such as hormones and proteins useful for a living body, or cells capable of detoxifying harmful substances can be retained stably for a prolonged period of time, resulting in exhibiting efficacy on prevention/treatment of endocrine/metabolic disease, hemophilia, bone disease or cancer by its administration to patients.

### BACKGROUND ART

Bioartificial organs are a device intended to prevent or treat diseases of patients by containing viable cells and biotissues and supplying metabolic function needed to patients, more concretely, biologically active factors such as hormones and proteins regulating the metabolic function in patients. As compared with living donor organ transplantation having problems of side effects of immunosuppressants, and demand and supply of donors, bioartificial organs can protect cells by immunoisolation membranes from the defense mechanism of a living body, and are excellent in terms of their applicability not only for allotransplantation but also for heterotransplantation without immunosuppressants.

The bioartificial organ can deal with therapy to any kinds of diseases by changing the kind of cells to be contained therein. For example, a bioartificial pancreatic islet contains insulin secreting cells, e.g. pancreatic islet cells, therein and is employed for supplying insulin hormones secreted from the pancreatic islet cells to a patient to normalize the blood sugar level. A blood coagulation factor-production type bioartificial organ contains liver cells producing the blood coagulation factor therein and is used for treatment of hemophilia causing blood coagulation disorder owing to insufficiency of VIII factor or XI factor. Further, a growth hormone production type bioartificial organ contains growth hormone (hGH) - secreting cells therein and is employed for medical treatment of pituitary dwarfism or the like caused by insufficient secretion of growth hormones. Also, addition of cells secreting respectively parathyroid hormones and erythropoietin to the bioartificial organ enables to treat for other diseases such as hypoparathyroidism and anemia.

The bioartificial organ is formed in a variety of shapes. Examples thereof are microcapsule shapes and macro-capsule shapes encapsulating cells with high molecular weight polymers. They are characterized in that the cells contained therein are protected from the defense mechanism of a living body by the firm crosslinking structure of the high molecular weight polymers and further the hormones or the like secreted from the cells are supplied to the living body by utilizing the molecular permeability of the high molecular weight polymers.

As a high molecular weight polymer to be used for a microcapsule type artificial organ or the like, polyvinyl alcohol (hereinafter, referred to as PVA) has been drawing attention in recent years. PVA has been conventionally used in the field of foods and medicines, and more concretely, it has been used as surgical suture threads, containers to be brought into contact with foods and the like. Also, a technique to use PVA for artificial articular cartilage materials has been disclosed (e.g. Non-Patent Document No.1) and its safety has been evaluated highly in clinical fields (e.g. Non-Patent Document No.2).

PVA becomes gel by chemical or physical treatment and is formed in any shapes. As the chemical treatment, there may be generally employed a method of adding glutaraldehyde (a crosslinking agent) and hydrochloric acid (a catalyst) to an aqueous solution containing PVA (e.g.Non-Patent Document No.3), and a method of applying an aqueous solution containing PVA to a glass plate or the like and immersing the glass plate in an aqueous Na₂SO₄/KOH solution (e.g. Non-Patent Document No.4) or the like. Also, the physical treatment used may include generally a method of quenching an aqueous solution containing PVA at -80°C for gelation. However, in the case of using PVA as a high molecular weight polymer for microcapsule type artificial organs, necessity of these chemical or physical treatments becomes a problem.

Concretely, there is a method of producing a bioartificial organ by chemically treating PVA to make a PVA gel, forming a bag-like PVA gel membrane and placing cells into the bag. However, with respect to this method, the cells may possible be damaged by sealing treatment of the bag or crosslinking agents remaining in the PVA gel membrane, or the cell necrosis takes place owing to the coagulation of the cells in the bag, which inevitably results in decrease of hormone secretion due to decreased number of viable cells or reduction of the biological activity.

Since no chemical agent is used and cells can be encapsulated dispersedly in PVA gel, the physical treatment is preferable because of no risk of cell necrosis through coagulation or the like, however, there is a possibility that cells may possibly be broken at the time of quenching at -80°C.

It is desirable for cells contained in bioartificial organs to stably supply a biologically active factor exhibiting metabolic function needed for patients. Accordingly, although PVA is a high molecular weight polymer suitable for such microcapsule type bioartificial organ owing to its properties, its application technique has not yet been established. Therefore, any bioartificial organ just like the present invention which utilizes the advantageous properties of PVA and in which cells can be stably retained in PVA gel has never been known yet.
Non-Patent Document No. 1:
   Kobayashi M., et al., Preliminary study of polyvinyl alcohol-hydrogel (PVA-H) artificial meniscus, Biomaterials, 2003, vol. 24, p639-647
Non-Patent Document No. 2:
   C.C.Demerlis et.al, Review of the oral toxicity of PVA, Food and Chemical Toxicology, 2003, vol. 41, p319-326
Non-Patent Document No. 3:
   Krystyna Burczak et. al, Long-term in vivo performance and biocompatibility of PVA hydrogel macrocapsules for hybrid-type artificial pancreas, Biomaterials, 1996, vol. 17, p2351-2356
Non-Patent Document No. 4:
   Tai-Horn Young et al., Assessment and modeling of PVA bioartificial pancreas in vivo, Biomaterials, 2002, vol. 23, p3495-3501

### DISCLOSURE OF THE INVENTION

An object of the present invention is to provide a cellular preparation useful for pharmaceutical compositions for human beings and animals. More particularly, the present invention aims to provide a cellular preparation wherein cells capable of secreting biologically active factors such as hormones and proteins useful for patients can be retained stably for a prolonged period of time in PVA which is a proper material as a high molecular weight polymer to be used for bioartificial organs., and a method for preparing such cellular preparation. Further, another object of the present invention is to provide a method for prevention and treatment of endocrine/metabolic disease, hemophilia, bone disease or cancer by administration of the cellular preparation to patients.

The inventors of the present invention have studied extensively to accomplish the above-mentioned aims and have found that the death ratio and the damage of cells in the PVA gel can be reduced and the cells can be stably retained in the PVA by previously treating PVA with a cell preservative, for example, Euro-Collins solution, Cell Banker or UW solution, and then mixing the PVA with cells to cause gelation of PVA. More practically, it was found possible to obtain a cellular preparation capable of suppressing decrease of the survival ratio of cells in PVA and deterioration of secretion ability of biologically active factors and further supplying hormones or proteins stably for a long duration to patients by at first producing a PVA-cell preservative mixed solution through dissolution of powder PVA into a liquid cell preservative, dispersing the cells in the resulting solution, quenching the cells in the mixed solution and effecting gelation of the PVA. The inventors of the present invention have made further studies based on these findings and consequently have accomplished the present invention.

That is, the invention relates to
(1) a cellular preparation comprising cells in polyvinyl alcohol mixed with a cell preservative;
(2) the cellular preparation as described in the above (1), wherein the cell surface is further coated with an extracellular matrix;
(3) the cellular preparation as described in the above (1) or (2) further containing a growth factor;
(4) the cellular preparation as described in any one of the above (1) to (3), wherein the cells are one or two or more kinds of cells selected from the group consisting of pancreatic islet cells, pancreatic endocrine cells, hepatocytes, anterior pituitary cells, growth hormone-secreting cells, osteocytes, thyroid hormone-secreting cells, and parathyroid hormone-secreting cells;
(5) the cellular preparation as described in any one of the above (1) to (4), wherein the cells are treated with a cell preservative;
(6) the cellular preparation as described in any one of the above (1) to (5), wherein the cells are transformed cells;
(7) the cellular preparation as described in any one of the above (1) to (6) having a sheet-like, plate-like, rod-like, tubular, or bead like shape;
(8) the cellular preparation as described in any one of the above (1) to (7), wherein the cell preservative solution is Euro-Collins solution, Cell Banker, or UW solution;
(9) the cellular preparation as described in any one of the above (1) to (8), which is transplanted subcutaneously, intramuscularly, or intraabdominally to mammals;
(10) a method for prevention and treatment of endocrine or metabolic disease, comprising using the cellular preparation according to any one of the above (1) to (9);
(11) the method for prevention and treatment of endocrine or metabolic disease as described in the above (10), wherein the endocrine metabolic disease is diabetes or pituitary dwarfism;
(12) a method for prevention and treatment of hemophilia, which comprises using the cellular preparation according to any one of the above (1) to (9);
(13) a method for prevention and treatment of bone disease, which comprises using the cellular preparation according to any one of the above (1) to (9);
(14) a method for prevention and treatment of cancer, which comprises using the cellular preparation according to any one of the above (1) to (9);
(15) a method for prevention and treatment of hepatic insufficiency or inborn errors of metabolism, which comprises using the cellular preparation according to any one of the above (1) to (9);
(16) a method for preparing a cellular preparation, which comprises mixing a cell preservative with a polyvinyl alcohol, and mixing cells with the polyvinyl alcohol, and gelling the resulting cell-containing polyvinyl alcohol; and
(17) the cellular preparation as described in any one of the above (1) to (9), which is a medicine for human or animal use.

### BRIEF DESCRIPTION OF THE DRAWINGS

Fig. 1 is a schematic view showing a production method of a sheet-like pancreatic islet cell preparation.
Fig. 2 is a graph showing test results of the recovery ratio of viable cells of Production Example 1, Comparative Example 1, or free pancreatic islet cells after 1 day-culture.
Fig. 3 is a graph showing test results of the insulin content in Production Example 1, Comparative Example 1, or free pancreatic islet cells after 1 day-culture.
Fig. 4 is a graph showing test results of insulin secretion with the lapse of time in Production Example 1, Comparative Example 1, or pancreatic islet cells.
Fig. 5 is a microphotograph of pancreatic islet cells contained in Production Example 1 or Comparative Example 1 by observation with the lapse of time.
Fig. 6 is a graph showing test results of insulin secretion of Production Example 2 or free pancreatic islet cells.
Fig. 7 is a graph showing the survival period of transplanted mouse group and diabetic mouse group.
Fig. 8 is a graph showing the change in blood sugar level of transplanted mouse group, diabetic mouse group, and normal blood sugar mouse group.
Fig. 9 is a graph showing the change in body weight of transplanted mouse group, diabetic mouse group, and normal blood sugar mouse group.
Fig. 10 is a graph showing the change in BUN (blood urea nitrogen) level of transplanted mouse group,diabetic mouse group, and normal blood sugar mouse group.
Fig. 11 is a graph showing the change in creatinine level of transplanted mouse group, diabetic mouse group, and normal blood sugar mouse group.
Fig. 12 is a graph showing the change in 24-hours urine of transplanted mouse group, diabetic mouse group, and normal blood sugar mouse group.
Fig. 13 is a graph showing the change in 24-hours urine glucose excretion of transplanted mouse group, diabetic mouse group, and normal blood sugar mouse group.
Fig. 14 is a graph showing the change in urine ketone level of transplanted mouse group, diabetic mouse group, and normal blood sugar mouse group.
Fig. 15 is a graph showing the change in 24-hours urine albumin excretion of transplanted mouse group, diabetic mouse group, and normal blood sugar mouse group.
Fig. 16 is a graph showing the change in 1,5-AG level of transplanted mouse group, diabetic mouse group, and normal blood sugar mouse group.

In the above Fig., the reference numeral 1 represents a pancreatic islet cells; 2 represents a PVA+EC solution; 3 represents PVA+EC gel; 4 and 6 represent mesh sheets; 5 and 7 represent glass plates; and 8 represents a sheet-like pancreatic islet cell-containing cellular preparation.

### BEST MODE FOR CARRYING OUT THE INVENTION

The present invention is characterized in that a cellular preparation contains PVA mixed with a cell preservative and contains cells producing/secreting biologically active factors such as hormones and proteins useful for patients in the PVA.

The PVA to be used in the present invention is not particularly limited without departing from the purpose of the invention and is preferable to have purity enough to be used, for example, corneal protectors/moisturizers in eye drops, medical materials such as intestinal adhesion preventing membranes, and food contact materials such as food wrapping films. It may also include commercialized products, and freeze-dry products obtained by producing polyvinyl acetate from vinyl acetate followed by hydrolysis. In the case of producing the PVA, the production method and the freeze-drying method may be carried out according to the known conventional methods. The PVA has preferably an average molecular weight of about 5,000 to 16,600 and a saponification degree of about 85% or higher.

The PVA is preferably a fine granular freeze-dried product which is easily suspended in a solution such as physiological saline and phosphate buffers

The PVA mixed with a cell preservative means PVA containing the cell preservative in the PVA. The cell preservative to be used in the present invention is not particularly limited and may include solutions to be employed generally for preservation of animal cells and commercialized preservation solutions, for example, Euro-Collins solution, Cell Banker (code 630-01601: Wako Pure Chemical Industries, Ltd. Osaka, Japan) and UW solution (ViaSpan: Bristol-Myers Squibb Company, USA). They may be commercialized ones or compositions prepared in laboratories. In the case of preparation in laboratories, it is preferable to carry out the preparation by adding and mixing each component in distilled water and disinfecting the obtained solutions by filtration with a filter.

For example, the composition of the Euro-Collins solution is shown in the following Table 1.

**Table 1**

| Component | g/L |
|---|---|
| D-glucose | 35.0 |
| K₂HPO₄ | 7.4 |
| KH₂PO₄ | 2.05 |
| KCl | 1.12 |
| NaHCO₃ | 0.84 |
| Nicotinamide | 1.22 |

Also, the composition of the UW solution (ViaSpan) is shown in the following Table 2.

**Table 2**

| Component | g/L |
|---|---|
| Pentafraction (note 1) | 50 |
| Lactobionic acid | 35.83 |
| Potassium dihydrogen phosphate | 3.4 |
| Magnesium sulfate | 1.23 |
| Raffinose | 17.83 |
| Adenosine | 1.34 |
| Allopurinol | 0.136 |
| Total glutathione | 0.922 |
| Potassium hydroxide | 5.61 |
| Sodium hydroxide | Adjusted to pH 7.4 |
| Water for injection | q.s. |

| | |
|---|---|
| Note 1: US Patent NO. 4,798,824 | |

The PVA containing a cell preservative in the PVA can be produced, for example, by mixing PVA with a cell preservative. The resulting PVA containing the cell preservative is desirably aseptic. The method of mixing PVA and a cell preservative is not particularly limited, however, there can be, for example, exemplified a method of suspending a commercialized granular PVA in distilled water, etc. dissolving and sterilizing the resulting PVA suspension by heating and disinfecting (autoclaving or the like), and adding and mixing a cell preservative to and with the obtained aseptic aqueous PVA solution. The heating and disinfecting treatment and the aseptic treatment themselves may be carried out according to the known conventional methods.

Herein, PVA containing the cell preservative produced by any means other than the above-mentioned mixing means may be called as PVA treated with the cell preservative and therefore, it should go without saying that PVA treated with the cell preservative is also included within the scope of the present invention.

The content of PVA in the mixed solution (hereinafter, called as a PVA-cell preservative solution) obtained by mixing an aqueous suspended PVA solution and a cell preservative is generally about 2 to 5% by weight, more preferably about 2.5 to 3.5% by weight, and especially preferably about 2.5 to 3% by weight based on the PVA-cell preservative solution. It is because gelation of PVA is most easily caused in this concentration range and also PVA gel with preferable strength for encapsulating the cell in the cellular preparation of the present invention. If the concentration of the cell preservative to be used for preserving the cells is defined to be 1 time, the concentration of the cell preservative in the PVA-cell preservative solution is generally about 0.8 to 1 time, more preferably about 0.9 to 1 time, and especially preferably about 0.95 to 1 time as much. It is because if the concentration is out of this range, the cells cannot sometimes survive stably. Accordingly, in the present invention, it is preferable that a concentrated cell preservative solution with a concentration of about 2 to 10 times as high as that for normal cell preservative solution is prepared or purchased, and then properly mixed with the above-mentioned aqueous suspended PVA solution (e.g. 10 times high concentration cell preservative solution: aqueous PVA suspended solution = 1 : 9) to obtain a PVA-cell preservative solution containing the cell preservative in about 1 time concentration.

The cellular preparation of the present invention may further contain dimethyl sulfoxide (DMSO) and serum albumin for protecting the cells, antibiotics, etc. for inhibiting contamination of germ, and also vitamins such as nicotinamide for cell activity retention. Further, in the present invention, other additives which are generally allowed to add under Pharmaceutical Affairs Law, e. g. an agent for proving sustained release property, an isotonic agent, a pH adjustment agent, and the like may be supplemented. The addition method of these other additives to the cellular preparation is not particularly limited, however, it is preferable to add them to the above-mentioned PVA-cell preservative solution in aseptic manner at the time of production of the solution. The contents thereof are preferably within a range of not inhibiting the growth/survival of the cells contained therein and/or the secretion of biologically active factors by the cells, and without departing from the scope and true sprit of the present invention.

The PVA treated with the cell preservative is produced in aseptic manner as described above and therefore, the cellular preparation of the present invention is seldom to be contaminated with germ and possible to be preserved at room temperature (about 15 to 35°C) for a long period of time.

The cells to be used for the present invention may include pancreatic islet cells, pancreatic endocrine cells, hepatocyte, anterior pituitary cells, growth hormone-secreting cells, osteocytes, thyroid hormone-secreting cells, and parathyroid-hormone-secreting cells. These cells are preferably derived from a mammal such as human being, pig, rat, and mouse, and those which produce/secret biologically active factors such as hormones and proteins effective for patients. Selection of cell types is preferably determined depending on the type of diseases of patients receiving the cellular preparation. For example, pancreatic islet cells or pancreatic endocrine cells, etc., producing insulin are preferable for diabetic patients, etc.; hepatocytes, etc., producing blood coagulation factors for hemophiliac patients, etc.; anterior pituitary cells and growth hormone-secreting cells, etc., producing growth hormones for pituitarydwarfismpatients, etc . ; and osteocytes producing bone morphogenetic proteins (BMP) for patients suffering from bone diseases such as bone fracture. Transformed cells capable of producing a large quantity of tumor growth suppressing substances by genetic recombination may be used for cancer patients. Further, cells having functions of selectively metabolizing and detoxifying harmful metabolites may be used for patients suffering from diseases which accumulates harmful metabolites due to hepatic insufficiency, renal insufficiency, or inborn errors of metabolism. In the case where a patient needs a plurality of biologically active factors since he suffers from a plurality of diseases, two or more kinds of the above-mentioned cells may be combined. Biologically active factors efficacious to the above-mentioned patients can be provided since the present invention contains these cells. Accordingly, use of the cellular preparation of the present invention makes it possible to prevent or treat various diseases whose remediation is possible, through supply of biologically active factors or detoxification of harmful metabolites.

The above-mentioned cells to be employed in the present invention may be an established cell line for laboratories or cells isolated from tissues of a living body, however, they are preferably differentiated, non-dividing cells since differentiated, non-dividing cells are more capable of producing /secreting target hormones and proteins than non-differentiated, dividing cells.

The isolation method of the cells from living tissues is not particularly limited, and known conventional techniques may be employed. For example, the known methods include a method of extracting tissues by proper means, treating the tissues with Dispase, EDTA, or the like and then with trypsin to finally isolate single cells.

In the case where the cells used in the present invention are pancreatic islet cells, isolation of the cells from living tissues may be carried out by a separation method through the known collagenase treatment, e.g. J. Adam Van Der Vliet et al., Transplantation, 45(2), p493-495 (1988).

In the case where the cells to be employed in the present invention are pancreatic endocrine cells, the isolation may be carried out, for example, according to the method described in Japanese Patent Application Laid-Open No. 2001-231548.

The isolated cells from the living tissues in the above-mentioned manner are preferably free from pathogens such as pathogenic virus.

It is preferable for the cells already established for laboratories and single cells obtained from living tissues to be cultured in a suitable media until they become confluent and to be employed as cells for the present invention after passage culture is repeated 2 or 3 times. For example, in the case where the cells to be employed in the present invention are pancreatic islet cells or pancreatic endocrine cells (hereinafter, referred to as pancreatic endocrine cells or the like), these cells are preferably subjected to passage culture in a CMRL-1066 culture medium supplemented with 10% fetal bovine serum (hereinafter abbreviated as FBS) and nicotinamide (Sigma, St. Louis, MO, USA). The passage culture cells are again isolated as single cells by the known method such as trypsin treatment or collagenase treatment to be used as cells for the present invention.

The cells to be employed in the present invention may be transformed cells into which gene coding peptides of biologically active factors such as hormones or proteins necessary for patients to be administered are introduced. Use of the transformed cells makes it possible to produce the target biologically active factors efficiently in a large amount. The base sequence of genes encoding peptides of such biologically active factors has already been opened, and such genes include, for example, genes available from American Type Culture Collection (ATCC) or from commercially easily available sources , and synthetic genes obtained by producing oligonucleotide probes from the known sequences and carrying out the synthesis using said probes by known conventional methods, for example, PCR amplification method and DNA synthesis method. For example, use of transformed cells containing a gene encoding tumor growth suppression proteins makes it possible to use the cellular preparation of the present invention for prevention and treatment of cancers.

Although there is not particular limitation to host cells into which a gene encoding the above-mentioned peptides of biologically active factors, and known conventional host cells to be used generally in the field of genetic recombination technology may be employed. As the host cells other than the above-mentioned pancreatic islet cells, pancreatic endocrine cells, hepatocytes, anterior pituitary cells, growth hormone-secreting cells, and osteocytes, there can be exemplified monkey COS-7 cells, Vero cells, Chinese hamster CHO cells (hereinafter abbreviated as CHO cell), dhfr gene-defective Chinese hamster CHO cells (hereinafter abbreviated as CHO(dhfr⁻)cell), mouse BALB/3T3 cells, mouse L cells, mouse AtT-20 cells, mouse C127 cells, mouse myeloma cells, rat GH3 cells, human HeLa cells, human FL cells, and the like.

The method for introducing the gene into the cells to be used in the present invention is not particularly limited and may be carried out by known conventional technique. For example, conventionally known methods of introducing the gene into recombinant expression vectors such as plasmids or viruses, and artificial vectors such as liposomes and microcapsules can be exemplified. In the case of using recombinant vectors, examples of applicable methods are competent cell method (J. Mol. Biol. , 53, p154 (1970)), DEAE dextran method (Science, 215, p166 (1982)), In-vitro packaging method (Proc. Natl. Acad. Sci. , USA, 72, p581 (1975)), Virus vector method (Cell, 37, p1053 (1984)), Microinjection method (Exp. Cell. Res., 153, p347 (1984)), Electroporation method (Cytotechnology, 3, p133 (1990)), Potassium phosphate method (Science, 221, p551 (1983)), Lipofection method (Proc. Natl. Acad. Sci., USA, 84, p7413 (1987)), Protoplast method (Japanese Patent Application Laid-Open No. 63-2483942, Gene, 17, p107, (1982), Molecular & General Genetics, 168, p111 (1979)).

Although there is no particular limitation on the vectors for introducing their gene into the host cells, any expression vectors so long as it can express desired gene in the cells into which they are introduced and can produce efficiently the peptides of biologically active factors may be used. For example, animal viruses such as bacteriophage, e.g. λ phage, adenovirus, adeno-associated virus (AAV), retrovirus, poxvirus, herpes virus, herpes simplex virus, lentivirus (HIV), Sendai virus, Epstein-Barr virus (EBV), vaccinia virus, poliovirus, Sindbis virus, SV 40 , and also pA1-11, pXT1 , pRc/CMV, pRc/RSV, pcDNAI /Neo , and the like can be exemplified.

The cells to be employed in the present invention are preferably contained in the PVA by the following techniques.

Cells are added and mixed in the above produced PVA-cell preservative solution. The cells to be added and mixed are preferably treated previously with a cell preservative. Practically, the cells are preferably suspended previously in the cell preservative and recovered by centrifugation, and then mixed with the PVA-cell preservative solution. The number of the cells to be contained in the cellular preparation of the present invention cannot be generalized since it depends on the types and the extent of the diseases of patients to whom the cellular preparation is administered, and it is therefore preferable to determine the number of cells by a doctor. The number of cells is preferably about 1×10⁷ to 5×10⁷ cells/ml in the PVA-cell preservative solution. Adjustment of the number of the cells within the range makes the cells dispersed evenly in the PVA gel and the cells can stably survive for a long duration without inhibition of supply of oxygen and nutrients to the cells due to cell coagulation.

Also, in order to retain the cells to be employed in the present invention in the stable condition for optimum supply of the biologically active factors, the cellular preparation of the present invention may further contain mucopolysaccharides such as hyaluronic acid, chondroithin sulfuric acid, and dermatanic acid; extracellular matrix containing one or more substances such as erastin, collagen, and fibrin; and/or growth factors such as hepatocyte growth factor (HGF), vascular endothelial cell growth factor (VEGF), human basic fibroblast growth factor (bFGF), fibroblast growth factor (FGF), platelet-derived growth factor (PDGF), insulin-like growth factor (IGF), and growth hormone (GH). One or more kinds of these growth factors may be contained. The method for adding the extracellular matrix and/or growth factors in the present invention may be a method involving, for example, immersing the cells in a cell culture medium containing the extracellular matrix and the growth factor for forming the extracellular matrix on the surfaces of the cells before mixing the above PVA-cell preservative solution with the cells, or a method of previously adding the extracellular matrix and the growth factor in the PVA-cell preservative solution, or the like. The content of the above-mentioned extracellular matrix and/growth factor in the cellular preparation of the present invention is not particularly limited, however, it is within the range without inhibition of the cell retention and the secretion function of the biologically active factors and preferably in the range for causing effects on prolongation of the cell survival period and extension of the secretion period of the biologically active factors.

The cellular preparation of the present invention can be produced by mixing the PVA-cell preservative solution with the cells, then cooling the cell mixed solution (hereinafter, referred to as cell-containing PVA-cell preservative solution), and gelling the PVA . Cooling is preferably carried out by keeping the solution in a housing at a temperature as ultra low as about -20 to -80°C for about a half of a day to 3 days or in a housing at a temperature as ultra low as about -80°C for about 18 to 30 hours.

In the present invention, since the cell preservative is previously mixed with the PVA, even if the quenching is carried out at the above-mentioned temperature, necrosis or damage of the cells can be suppressed. Moreover, the cells can be contained in the PVA gel in dispersed manner and coagulation of the cells can be prevented.

Owing to the gelation of the PVA, the cellular preparation of the present invention may be formed in various shapes such as sheet, plate, board, rod, tube, or beads. For example, the above-mentioned cell-containing PVA-cell preservative solution is applied to a glass plate and cooled together with the glass plate to produce a cellular preparation in the form of a thin sheet. The cellular preparation with a desired thickness can be produced by properly adjusting the application dose of the cell-containing PVA-cell preservative solution per unit surface area of the glass plate and it is generally about 100 to 300 µl/mm².

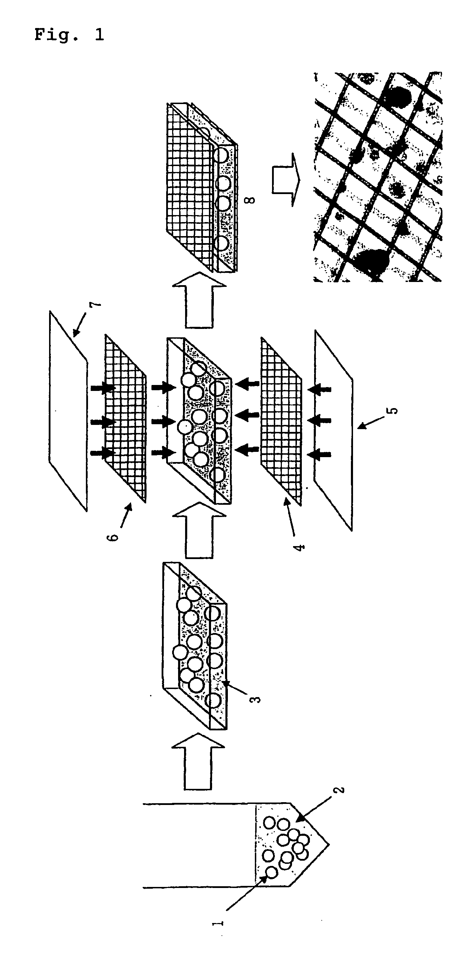

The cellular preparation of the present invention may be used in combination with a reinforcing material for reinforcement and/or simplicity in the handling. For example, in the case where the cellular preparation is formed in the form of a thin sheet-like shape, to reinforce it and to simplify in its handling, gelation is preferably carried out by fixing the cell-containing PVA-cell preservative solution in a mesh sheet made of a resin or the like. Specifically, in Fig. 1, for example, a mesh sheet (4) made of PET (polyethylene terephthalate) is put on a glass plate (5) and the above-mentioned cell-containing PVA-cell preservative solution is applied to the mesh sheet (4). Another mesh sheet (6) is put thereon and further another glass plate (7) is put thereon to sandwich the cell-containing PVA-cell preservative solution and the mesh sheets (4) and (6) with glass plates (5) and (7). While being kept as it is, the assembled body is cooled to about -20 to -80°C to form it into a sheet-like gel. The above-mentioned mesh sheet is preferably immersed previously in the PVA-cell preservative solution.

The above-mentioned reinforcing material, e.g. the mesh sheets, is preferably materials which are safe to living body, i.e. un-decomposable in vivo and excellent in biocompatibility, such as PET, and the like,. It is because if the mesh sheets in the cellular preparation of the present invention are decomposed in vivo, the cellular preparation accretion to living tissues takes place not only to make the cellular preparation sometimes difficult to maintain the efficacy for a long duration but also to make it harmful for living body. Of course, it is no need to say that the reinforcing materials which are harmless in a living body even they are decomposed in vivo are also usable in the present invention.

The cellular preparation in the frozen state is preferably administered to a living body after the cells in the frozen state are activated. The activation of the cells in the frozen cellular preparation is preferably carried out by thawing the cells and culturing them again in a proper culture medium. To be more specific, such activation is carried out by quickly immersing the frozen state cellular preparation in a cell culture medium of about 37°C, such as CMRL - 1966, and culturing the thawed cells again for about 24 hours in a new culture medium. In the case where the cellular preparation contains DMSO, it is preferable that the unfrozen cellular preparation is washed with a new cell preservation solution, e.g. an UW solution, etc. and further immersed in the UW solution at about 4°C for about 24 hours to remove DMSO from gel. It is no need to say that the cellular preparation which is thawed and made free from DMSO in the above-mentioned manner is also included in the cellular preparation of the present invention.

The administration manner of the cellular preparation obtained by the above-mentioned method to a living body differs depending on the type, the affected part, and severity of diseases of patients to be administered, and it is therefore preferably determined by a doctor. Hereinafter, a preferable administration manner will be described.

The subjects to which the cellular preparation of the present invention are administered include human being and also a mammal other than human being, e.g. dog, cat, monkey, rabbit, mouse, and the like.

As described above, the cellular preparation of the present invention can be administered in the form suitable to the application site of a living body. Also, the cellular preparation of the present invention may be transplanted to the application site in a direct contact manner to the affected tissues, and also it can be transplanted in the subcutaneous tissue or the muscle with relatively slight invasion. For example, the cellular preparation in tubular shape is cut into small fragments and transplanted in the subcutaneous tissue by using a surgical suture needle, or the cellular preparation can be injected in the muscle and subcutaneous tissue by filling an injector with the cellular preparation. The cellular preparation is easy to be recovered from the administration site. In the case where the cellular preparation is transplanted in the subcutaneous tissue and muscle, if the vascular distribution in the transplantation site is sparse and supply of oxygen and nutrients for growth and survival of the cells is supposed to be difficult, transplantation may be carried out together with known proper angiogenesis inducers. The angiogenesis inducers may be added previously in the cellular preparation or may be administered separately.

The dose of the biologically active factors such as hormones and proteins to be supplied to a patient from the cellular preparation of the present invention may properly be set by determination by a doctor in consideration of the secretion of the biologically active factors of the cells to be employed and the change of the cell survival ratio during the transplantation. For example, if a patient suffers from diabetes, the insulin secretion of the cells to be used, e. g. pancreatic islet secretion cells, is previously measured in vitro and depending on the insulin secretion, the number of the cells, administration period, and change of the survival ratio of the cells, the insulin supply amount of the cellular preparation of the present invention can be determined. Since the cellular preparation of the present invention can stably preserve the cells contained therein for a long duration, deterioration ratio of the secretion of the biologically active factors with the lapse of time is low. Accordingly, the present invention is suitable to supply the biologically active factors needed for patients stably for a long duration, resulting in decreased frequency of the transplantation of the cellular preparation.

The secretion of the biologically active factors of the cells to be used in the cellular preparation of the present invention may quantitatively be determined according to the known quantitative determination methods suitable for measurement of such biologically active factors. For example, a quantitative determination method of insulin in the case where the cells used are pancreatic islet secretion cells will be described in detail in the following Examples.

### EXAMPLES

The invention will be illustrated more specifically by way of Examples. However, it is not intended that the invention be limited to Examples. In Examples, % means % by weight unless otherwise specified.

### Example 1

### (Production Example 1) Production of sheet-like cellular preparation containing pancreatic islet cells

### (1) Preparation of pancreatic islet cells

Pancreatic islet cells were isolated from Wister rats with 8 to 10 week-old and 300 to 350 g weight (Shimadzu animal Co. Ltd. Kyoto, Japan) by the following method.

At first, 10 ml of Hank's solution containing type I collagenase (Sigma, St. Louis, MO, USA, 350 U/mg) and type XI collagenase (Sigma, St. Louis, MO, USA, 2,200 U/mg) 800 U/ml and 1500 U/ml, respectively, was injected to the pancreatic ducts of rat extracted pancreas and subjected to enzyme treatment at 37°C for 15 to 20 minutes. Cooled Hank's solution was added to stop the enzyme reaction. The pancreas tissues were dispersed with a pipette and centrifuged to recover tissue pellets, and then the tissue pellets were washed two times with Hank's solution. The centrifugation was carried out at 1,000 rpm for 1 minute. The tissue suspension was filtered through an 800 µm size mesh sheet. The filtrate was recovered in a 50 ml conical tube and centrifuged (1,500 rpm for 3 minutes) to obtain tissue pellets. The pellets were suspended in Hank's solution containing 27% dextran (Sigma, St. Louis, MO, USA, MW 70.000) and centrifuged by discontinuous density-gradient centrifugation. In this case, the lowermost layer was controlled to be 27% dextran (density 1.094 g/ml), and the upper layer was made into two layers of 23% dextran (density 1.081 g/ml) and 11% dextran (density 1.041 g/ml). The discontinuous density-gradient centrifugation was carried out at 400 rpm for 4 minutes and further at 1,700 rpm for 10 minutes. After centrifugation, the pancreatic islet cells-containing fraction was sampled in the interface between two uppermost layers with a Pustule pipette. The obtained pancreatic islet cells were washed two times with CMRL-1066 culture medium (1,000 rpm for 3 minutes).

### (2) Production of PVA-Euro-collins solution

PVA 3 g (manufactured by Gunse Co.: PVA-180H, Lot37435) was suspended in distilled water 75 ml and then subjected to heating dissolution and sterilization treatment through autoclaving (121°C, 15 minutes) two times. Ten times concentration of Euro-Collin solution (hereinafter abbreviated as EC solution) 10 ml, DMSO 5 ml, FBS 10 ml, and nicotinamide 0.122 g were aseptically added to and mixed with the obtained aseptic aqueous PVA solution, thereby to obtain an aseptic 3% PVA-EC solution 100 ml. The above-mentioned 10 times concentration of EC solution was prepared by admixing the components shown in Table 1 in distilled water 100 ml, and filtered and disinfected through 0.22 µm mesh. Thereafter, it was preserved at a room temperature.

### (3) Cell Mixing

The pancreatic islet cells obtained in (1) were cultured for 24 hours in a CMRL-1066 culture medium containing 10% FBS and nicotinamide 1.22 g/L in a carbon dioxide gas incubator (5% CO₂, 37°C). The culture solution containing 1,000 pancreatic islet cells was placed in a 1.5 ml centrifugation tube and centrifuged at 800 rpm for 1 minute. Cell Banker (code 630-01601: Wako Pure Chemical Industries, Ltd. Osaka, Japan) 0.1 ml was added to the obtained cell pellets and the cells were suspended, and then kept still for 5 minutes. The cells were recovered by centrifugation again. The recovered cells were suspended in the aseptic 3% PVA-EC solution prepared in (2) 100 µl to obtain a pancreatic islet cells-containing PVA-EC solution.

### (4) Formation of sheet

Two mesh sheets with 10 mm × 10 mm square made of PET (manufactured by Gunse Co.: TNO 60 SS) were previously immersed in an aseptic 3% aqueous PVA solution for 5 minutes, and one of the sheet (4) was put on a glass plate (5). The pancreatic islet cells-containing PVA-EC solution obtained in (3) was evenly applied to the above-mentioned mesh sheet (4), and another mesh sheet (6) was put further thereon to obtain a three-layer sheet composed of mesh sheet (4)/pancreatic islet cells-containing PVA-EC solution/mesh sheet (6). Further another glass plate (7) was put thereon so as to obtain about 1 mm-thick three-layer structure sheet by applying pressure, and PVA was converted to a gel by keeping the assembled body at -80°C for 24 hours while being sandwiched between two glass plates (see Fig. 1). (5) Thawing of pancreatic islet cellular preparation in sheet form and activation of cells

The frozen sheet-like pancreatic islet cellular preparation obtained in (4) was thawed quickly in CMRL-1066 culture medium at 37°C. The thawed sheet-like pancreatic islet cellular preparation was immersed in a UW solution 5 ml (ViaSpan: Bristol-Myers Squibb Company, USA) for 5 minutes. This procedure was repeated 3 times to remove DMSO contained in the sheet. The sheet was further immersed in a UW solution 5 ml at 4°C for 24 hours. The preparation after the immersion was washed with CMRL-1066 culture medium twice and then cultured in CMRL-1066 culture medium for 24 hours (37°C, 5% CO₂) to obtain a pancreatic islet cellular preparation containing activated cells (Production Example 1) in a sheet from. Production Example 1 was employed for the following tests. An aseptic 3% aqueous PVA solution was prepared as described in (2) without using EC solution 10 ml, DMSO 5 ml, FBS 10 ml, and nicotinamide 0.122 g and a pancreatic islet cellular preparation produced from the solution in the form of sheet was employed as Comparative Example 1.

### (Test Example 1) Confirmation of cell stability in preparations

Cells of Production Example 1 and Comparative Example 1, and free pancreatic islet cells (200 cells) were cultured respectively in CMRL-1066 culture medium for 24 hours (5% CO₂, 37°C) . The number of the viable cells in Production Example 1 and Comparative Example 1, and the number of free pancreatic islet cells after 1-day culture were counted using a microscope to calculate the recovery ratio (%) of the viable cells. The recovery ratio was a relative value to 100 defined as the number of the cells before the culture.

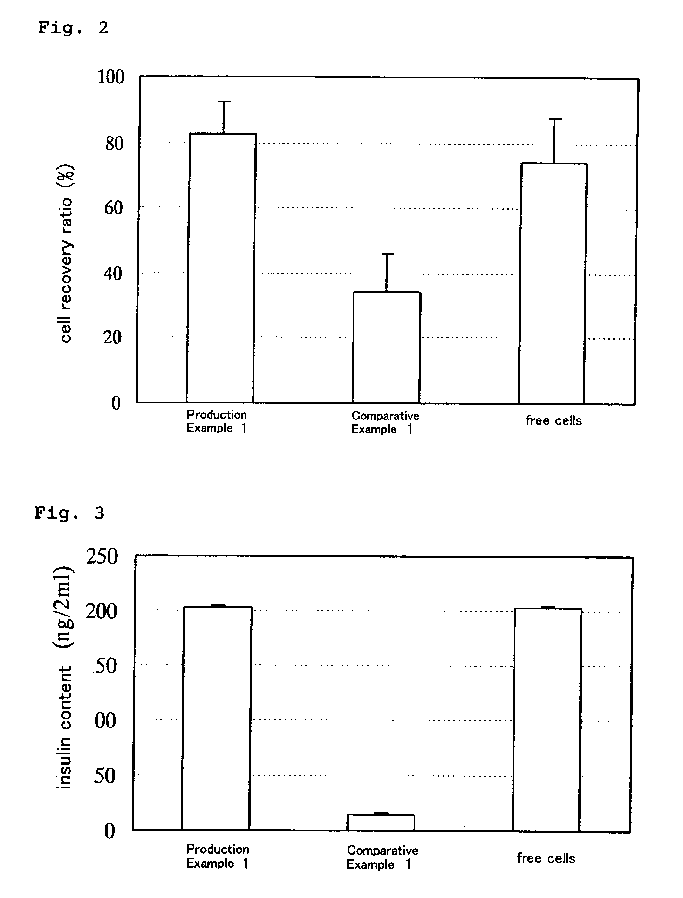

The results are shown in Fig. 2. The recovery ratio of the viable cells in Production Example 1 was 82%, which was extremely higher than that of Comparative Example 1 (about 35%), and the value was higher than that of the free cells.

### (Test Example 2) Confirmation of insulin content in preparations

Cells of Production Example 1, and Comparative Example 1, and pancreatic isolated islet cells (200 cells) were cultured respectively in CMRL-1066 culture medium for one day (5% CO₂, 37°C). The respective insulin amounts after 1-day culture were investigated. The cells of Production Example 1 and Comparative Example 1, and the free pancreatic islet cells were recovered in hydrochloric acid-EtOH and mashed respectively with a spatula, and the gel and the cells in the preparation were stirred and suspended in hydrochloric acid-EtOH with a vortex mixer (-20°C, 24 hours). The insulin concentrations in the respective cell suspensions were measured. Insulin was measured by using a commercialized insulin measurement ELISA kit (Lbis Insulin Kit, Shibayagi).

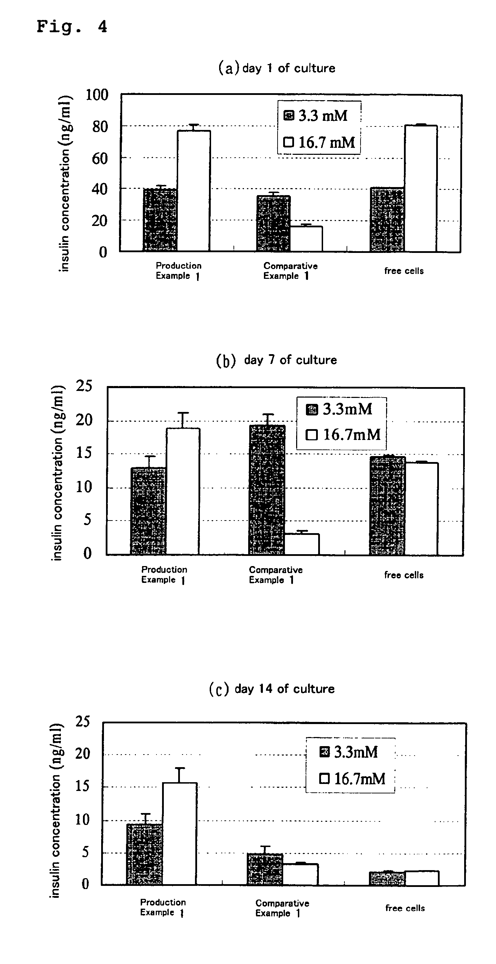

The results are shown in Fig. 3. The insulin content in Production Example 1 was about 205 ng/ml which was extremely higher than that of Comparative Example 1 (about 20 ng/ml), and the value was approximately the same as that of the free cells.

### (Test Example 3) Confirmation of insulin secretion of preparations and cell state

The cells of Production Example 1 and Comparative Example 1, and isolated islet cells (200 cells) were cultured in CMRL-1066 culture medium (5% CO₂, 37°C) and recovered after 1 day, 7 days, and 14 days. After the recovery, the recovered culture products were subjected to a glucose reaction test. The glucose reaction test was carried out as follows. At first the recovered cells of Production Example 1 and Comparative Example 1, and free pancreatic islet cells were cultured respectively in CMRL-1066 culture medium containing 3.3 mM glucose and 0.1% bovine serum albumin (BSA) for 1 hour (5% CO₂, 37°C) and then washed with glucose-free CMRL-1066 culture medium. This procedure was repeated again, and these cells were carried out in a CMRL-1066 culture medium containing 3.3 mM glucose for 1 hour and then the insulin concentration in each supernatant solution was measured. Next, culture was carried out in a CMRL-1066 culture medium containing 16. 7 mM glucose for 1 hour and then the insulin concentration in each supernatant solution was measured.

The results are shown in Fig. 4. In Fig. 4, (a) shows the amount (concentration) of insulin secretion after 1 day-culture; (b) shows the result after 7 days-culture; and (c) shows the result after 14 days-culture. In the case of Comparative Example 1, no increase in the insulin secretion depending on the glucose concentration was observed. On the contrary, in the case of Production Example 1, increase in the insulin secretion (concentration) depending on the glucose concentration was observed after 1 day-culture, 7 days-culture, and 14 days-culture. Moreover, in the case of free cells, no increase in the insulin secretion depending on the glucose concentration was observed after 7 days-culture, the increase was observed in the case of Production Example 1 even after 14 days-culture.

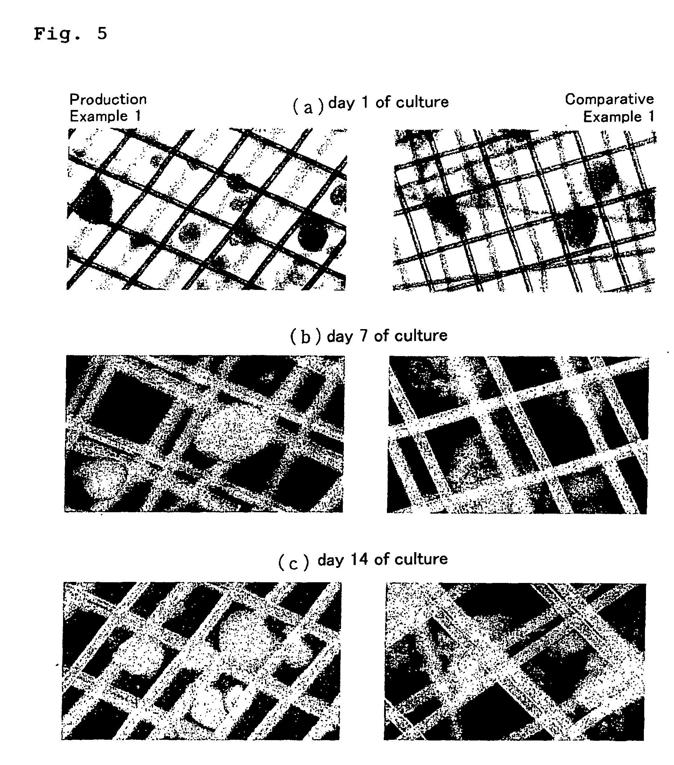

As a result of observation of cells contained in Production Example 1 and Comparative Example 1 by a microscope, the cells in Comparative Example 1 were found to be already destructed from the first day. On the other hand, no significant cell destruction for the cells of Production Example 1 was observed, and the cells were found to be relatively stable and were surviving even after 14 days (Fig. 5). In Fig. 5, (a) shows observation images of the cells after 1 day-culture; (b) after 7 days-culture; and (c) after 14 days-culture.

From the results, it was found that the islet cells stably survived in Production Example 1 and maintained the activity stably for a long duration and secreted insulin responding to the increase of glucose concentration. Accordingly, it was confirmed that previous addition of the EC solution to PVA suppressed cell necrosis and damage through quenching at the time of gelation of PVA, and as a result, it was proved that a cellular preparation with a high survival ratio of pancreatic islet cells contained in PVA and capable of retaining the insulin secretion and the responsiveness to increase in glucose concentration can be produced.

### Example 2

### (Production Example 2) Production of sheet-like cellular preparation containing pancreatic islet cells

### (1) Preparation of pancreatic islet cells

General anesthesia in Wister rats were carried out through diethyl ether inhalation and intraabdominal administration of Nembutal 1 mg/kg. After abdominal section, common bile duct was identified and clumped near the papilla of Vater by a non-toothed forceps. A small opening was made in the joined part of the cystic duct and a cannulation tube was inserted into the common bile duct, and fastened and fixed, and type XI collagenase (Sigma, St. Louis, MO, USA) equivalent to 1200 to 1500 u/Ml was injected to swell the pancreas. Without damaging, the whole pancreas was extracted, placed in a flask, immersed in a thermostatic bath of 37°C and digested in about 18 minutes. After digestion, the flask was shaken several times to destruct the pancreas and washed three times with Hank's solution containing 0.1% bovine serum albumin. After the cells were floated in the Hank's solution, the solution was filtered through an 850 µm-diameter mesh filter to recover the contents.

The islet separation was carried out using specific gravity gradation of Dextran 70. That is, the pancreatic islet cell components from which the supernatant solution was removed were stirred uniformly in Dextran with 1.094 specific gravity + Hank' s solution, and then Dextran with 1.094 specific gravity was slowly poured, and Dextran with 1. 081 specific gravity and Dextran with 1.041 specific gravity were slowly poured successively on the cell layer to form 4 layers. The layered sample was centrifuged at 400 rpm for 4 minutes and further at 1700 rpm for 13 minutes. The islet separated between the first layer and the second layer was recovered with a hand pick, washed and purified with a hand pick. After washing 4 times, the pancreatic islet cells were cultured overnight in an incubator under environments at 37°C, 5% CO₂, 95% air. The culture medium was CMRL 1066 + 10% inactivated fetal bovine serum + antibiotic-antimycotic solution (Gibco, BRL) + nicotinamide.

### (2) Preparation of pancreatic islet cellular preparation as sheet form

Using the culture solution of the pancreatic islet cells obtained in the above-mentioned manner, a sheet-like islet cellular preparation (Production Example 2) in which cells were activated was obtained similarly as described in (2), (3), (4), and (5) in Example 1.

### (Test Example 4) Insulin secretion of preparations

The above obtained islet cellular preparation (Production Example 2, after 1 day from the production) and free islet cells were stimulated for each 1 hour by low concentration glucose (3.3 mM) and high concentration glucose (16.7 mM) in the same manner as Test Example 3, and the amount of secreted insulin was measured by ELISA.

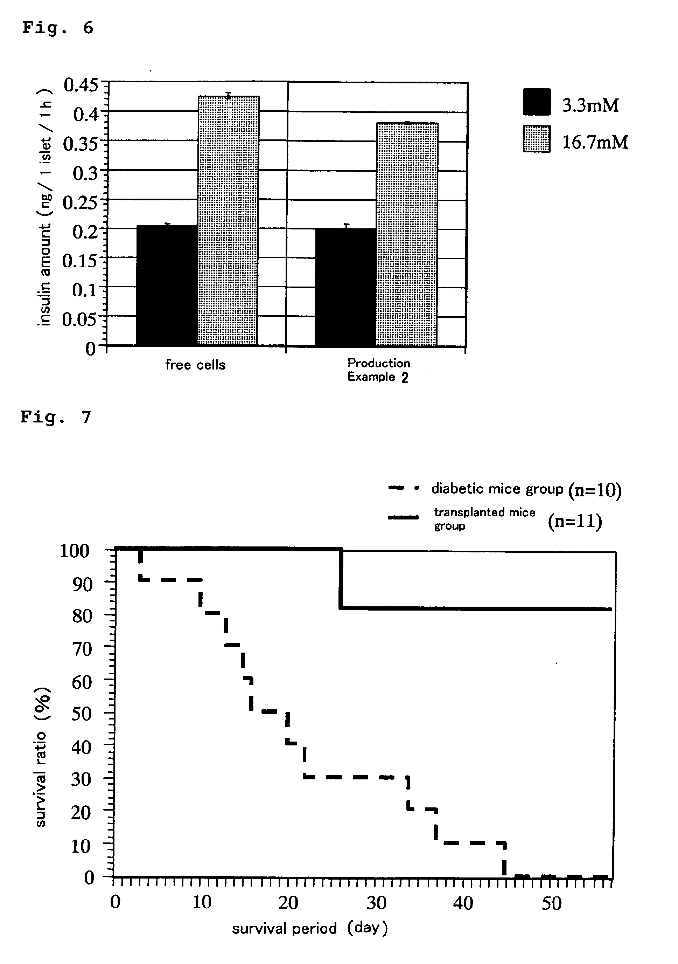

The results are shown in Fig. 6. As can be seen from Fig. 6, the islet cellular preparation according to the present invention was found to secrete insulin corresponding to the glucose stimulation and was not inferior to the free islet cells at the same period.

### (Test Example 5) Transplantation test

### (1) Production of diabetic mouse model

Streptozotocin 200 mg/Kg dissolved in a citric acid buffer (pH 4.5) was administered to C57BL/6 male mice of 8 to 10-week-old by intraabdominal administration. One week after the administration, blood sugar measurement was carried out three times, and mice showing blood sugar 350 mg/dl or higher two or more times were determined to be diabetic and used for the experiment. Mice without streptozotocin were used as a normal group of mice.

### (2) Transplantation

The transplantation of the islet cellular preparation was carried out as followings. That is, diabetic model mice (after 1 week or longer from diabetes development) were undergone abdominal section by median incision under ether anesthesia, and the islet cellular preparation (Production Example 2) obtained in Production Example 2 was kept in the abdomen and the abdominal wall was closed in two layers. These mice were defined as a transplanted mice group. Mice undergone transplantation of the preparation containing no pancreatic islet cells (produced in the same manner as in Comparative Example 1) were defined as a diabetic mice group.

### (3) Investigation of survival period

The survival period after the transplantation defined as 0 day was observed.

The results are shown in Fig. 7. It is apparent from Fig. 7 that a half or more of the diabetic mice group died within 4 weeks after the transplantation and no mouse could survive up to 8 weeks during the passage observation period. On the other hand, except two mice which showed continuously high blood sugar state and finally died among the group of the mice with the islet cells, others survived 8 weeks or longer (survival ratio up to 8 weeks: 0% vs. 81.8%; p < 0.001).

### (4) Blood sugar/weight measurement

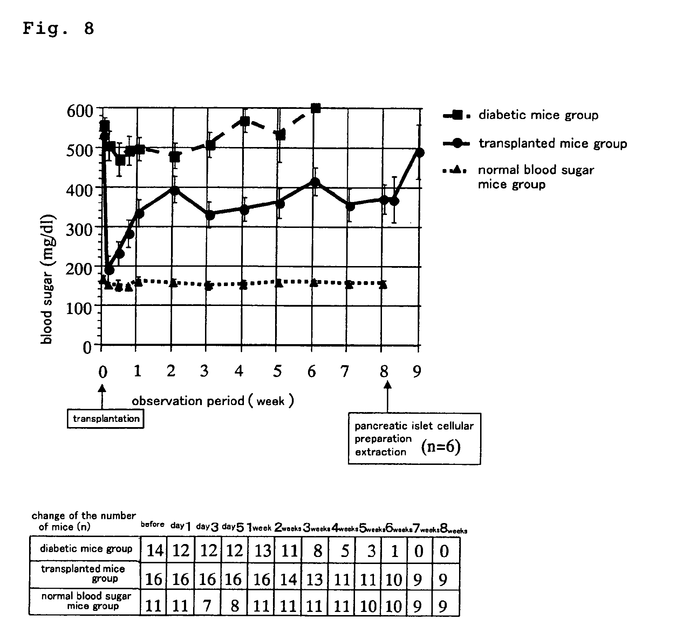

Both of the blood sugar and the weight were measured before the transplantation and after 1 day, 3 days, 5 days, and 7 days from the transplantation and thereafter measured once a week. The blood sugar fluctuation of each group was shown in Fig. 8. With respect to the transplanted group, many quickly showed decrease of the blood sugar immediately after transplantation to less than 300 mg/dl (13/16) and a majority of them were observed to be normal, however it was gradually increased (the period of keeping less than 300 mg/dl was 3 days to 7 weeks). However, the blood sugar increase was slight and the blood sugar level was significantly improved (p < 0.0001) as compared with that of the diabetic mouse group and the life recuperation up to 8 weeks from the transplantation was apparently improved. Among 9 mice observed up to 8 weeks, 6 mice were undergone extraction of the islet cellular preparation and among them 5 mice showed blood sugar increase again.

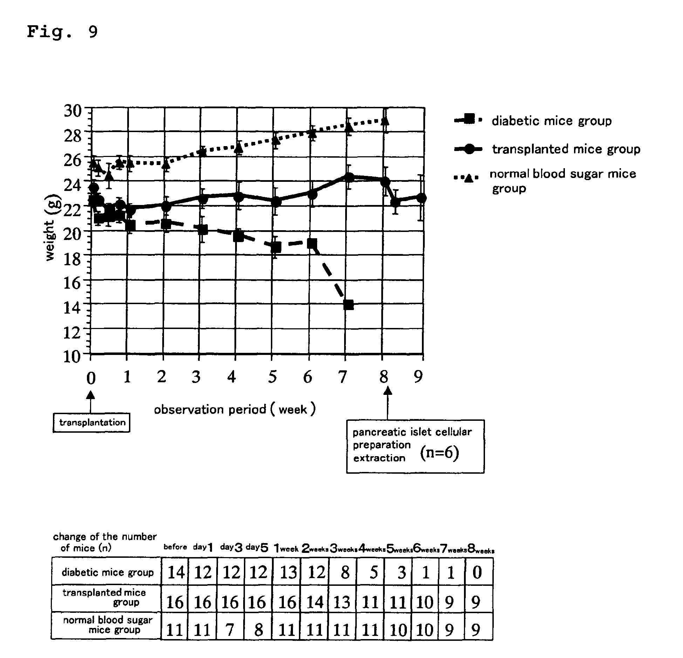

The average weight fluctuation of each group is shown in Fig. 9. In the group of the transplanted mice, the weight was decreased after surgical transplantation, and it is supposed to be due to the poor dietary, and no significant difference from the diabetic mice group was observed in the Repeated-Measure Analysis of Variance carried out up to 3 weeks after the transplantation. However, after 3 weeks from the surgical transplantation, the weight was turned to increase, and as compared with that in the first week when the average weight was most decreased during the passage, significant increase was observed after 3 weeks (p < 0.05) . Further, the average weight significantly exceeded the average weight of the diabetic mice group in 3 weeks and 4 weeks from the transplantation.

### (5) Measurement of kidney function

BUN (blood urea nitrogen) and creatinine were measured once a week before the transplantation and once a week after the transplantation. The pancreatic islet cellular preparation was extracted 8 weeks after the transplantation and thereafter the measurement was carried out until 1 week after the extraction.

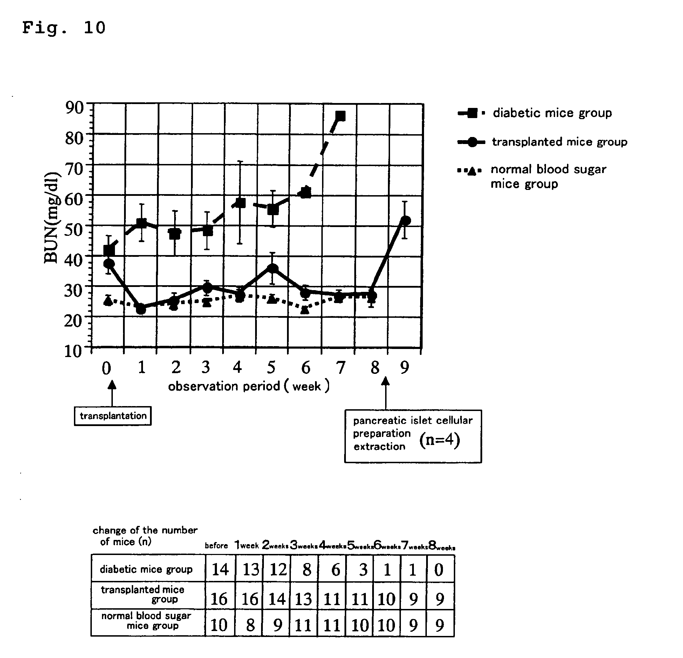

The fluctuation of BUN is shown in Fig. 10. The average ± SE of BUN of mice with normal blood sugar measured before the transplantation of the islet cellular preparation was 25.53 mg/dl ± 1.804 (20 to 38.9). Among the group of diabetic mice after 1 week from the administration of the streptozotocin, 41.3% showed BUN increase (24/58, diabetic mice group: 13/35 vs. transplanted mice group: 11/23, BUN > 35 mg/dl). Among 14 diabetic mice in the diabetic mice group, 13 mice showed high BUN value in the passage.

On the other hand, with respect to 16 transplanted mice in the transplantation mice group, 9 mice showing the BUN increase before the surgery, was found normalized after 1 week from the transplantation. The BUN value in average decreased after 1 week from the transplantation were approximately normal value and found significantly different from that of the diabetic mice group (p = 0.002).

Further, among 6 mice undergone the extraction of the pancreatic islet cellular preparation after 8 weeks from the transplantation, the BUN value measurement was carried out for 4 mice in one week after the extraction and all showed increase as compared with the value before extraction.

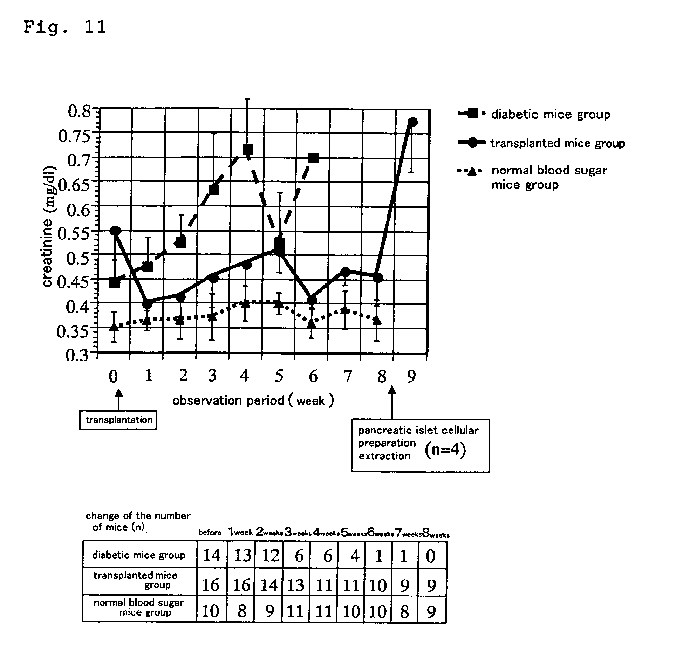

The change in creatinine level is shown in Fig. 11. The normal value of creatinine was 0.351 mg/dl ± 0.032 (0.16 to 0. 5). Almost a half of mice in the diabetic mice group showed as high as 0.5 mg/dl 1 week after the streptozotocin administration and all of the mice were found abnormal in the creatinine value 4 weeks after the administration. Further, after the creatinine increase, all of the mice were dead in several weeks and no mouse survived up to 10 weeks from the streptozotocin administration. Although 6 mice in the transplanted mice group showed the increase after implantation of the creatinine, the increase was slight and mice could survive during the observation passage. Although the creatinine average value in the transplanted mice group exceeded 0.5 mg/dl only 5 weeks after the transplantation, the value was constantly around 0.4 mg/dl in any period and was decreased significantly as compared with that of the diabetic mice group (p = 0.0478) . Further, similarly to the case of BUN, all mice showed increase again after the extraction of pancreatic islet cells.

### (6) Urine inspection

Urine was collected for 24 hours and the urine amount and 24-hour urine sugar excretion amount, 24-hour urine albumin excretion amount, and urine ketone were measured. All of the group of transplanted mice, the group of diabetic mice and the group of normal blood sugar mice were fed in free drinking and eating dietary environments for 24 hours, and the 24-hour urine was collected. Urine sugar was measured by Fuji DRI-CHEM system and the value calculated by multiplying the urine amount was defined to be the urine sugar excretion amount. The 24-hour urine albumin excretion amount was calculated by measuring the urine albumin concentration by ELISA (Albuwell M (Exocill)) and multiplying the 24-hour urine amount. The urine ketone was evaluated by using Urotape (Eiken) and evaluated by numeration of the color tone.

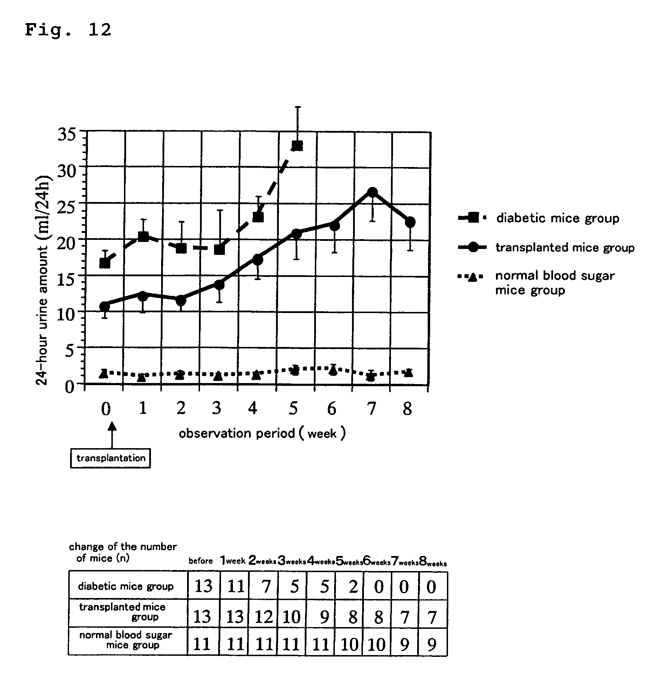

The change of 24-hour urine amount is shown in Fig. 12. The group of the transplanted mice did not show any significant difference, though slightly lower, from the group of diabetic mice.

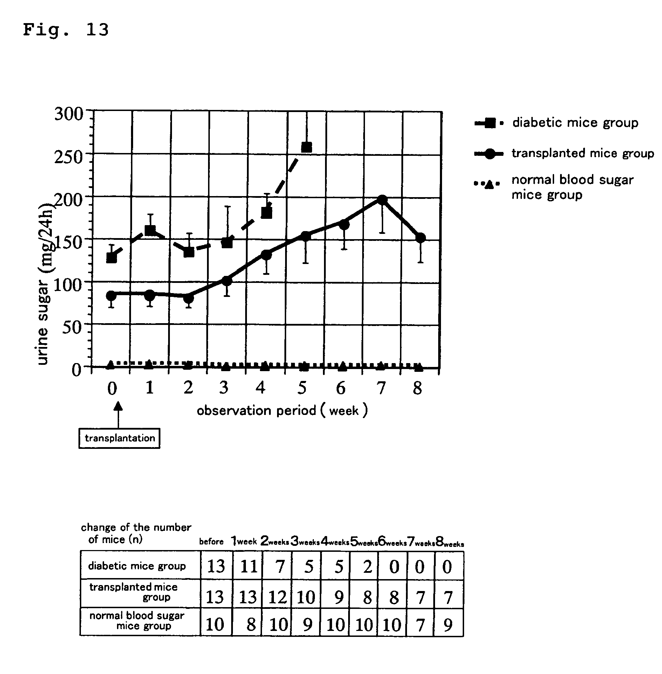

The change of 24-hour urine sugar amount is shown in Fig. 13. With respect to the urine sugar excretion amount, the group of the transplanted mice showed slightly lower value and had significant difference (p < 0.0452) to 3 weeks from the transplantation.

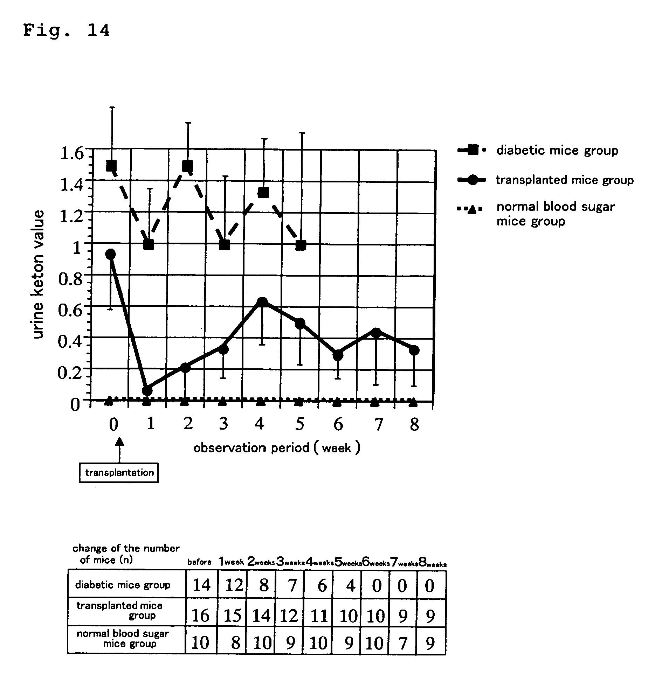

The change of urine ketone amount is shown in Fig. 14 . With respect to the urine ketone amount, both of the group of diabetic mice and the group of the transplanted mice showed increase before transplantation. In the group of the transplanted mice, the value was normalized 1 week after the transplantation, and thereafter, the value was decreased significantly as compared with that of the group of the diabetic mice (p = 0.0294).

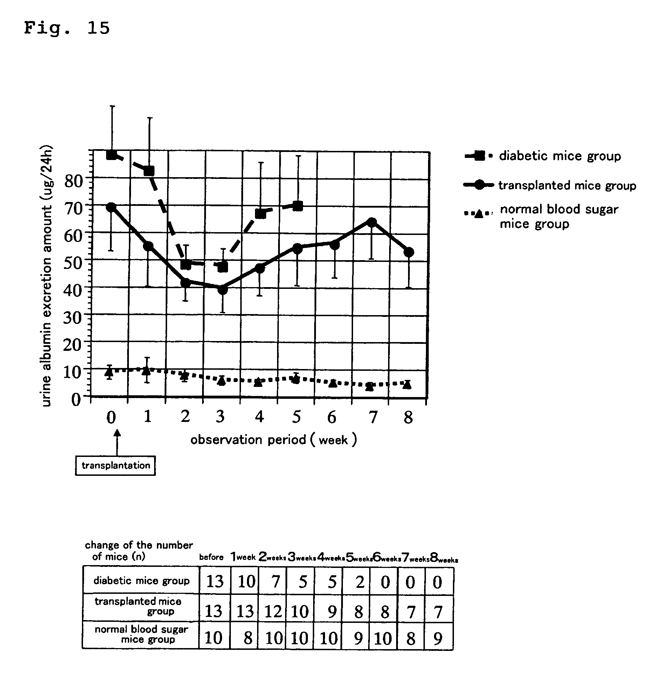

Further, the change of urine albumin amount is shown in Fig. 15. The urine albumin amount was lower in the group of the transplanted mice than in the group of diabetic mice, however no significant difference was found.

### (7) Measurement of 1,5-anhydro-D-glucitol (1,5-AG)

To investigate the grade of the diabetes, 1, 5-AG measurement was carried out. The 1, 5-AG is a polyol having extremely similar structure to that of glucose and mainly taken from food and absorbed and accumulated abundantly in a living body and reabsorbed in the kidney. It has competitive action against glucose, and in diabetes, the reabsorption of the urine sugar AG is inhibited to increase excretion of AG in urine and decrease AG in blood. Accordingly, 1,5-AG is very sensitively reacted to the dynamism of glucose and therefore, 1,5-AG can be a marker reflecting real time condition of diabetes. Blood 25 µl of the group of transplanted mice, the group of the diabetic mice, and the group of the mice with normal blood sugar was sampled from the tail vein once a week after the transplantation and measured by 1,5-AG kit for animal use (Nippon Kayaku Co., Ltd.)

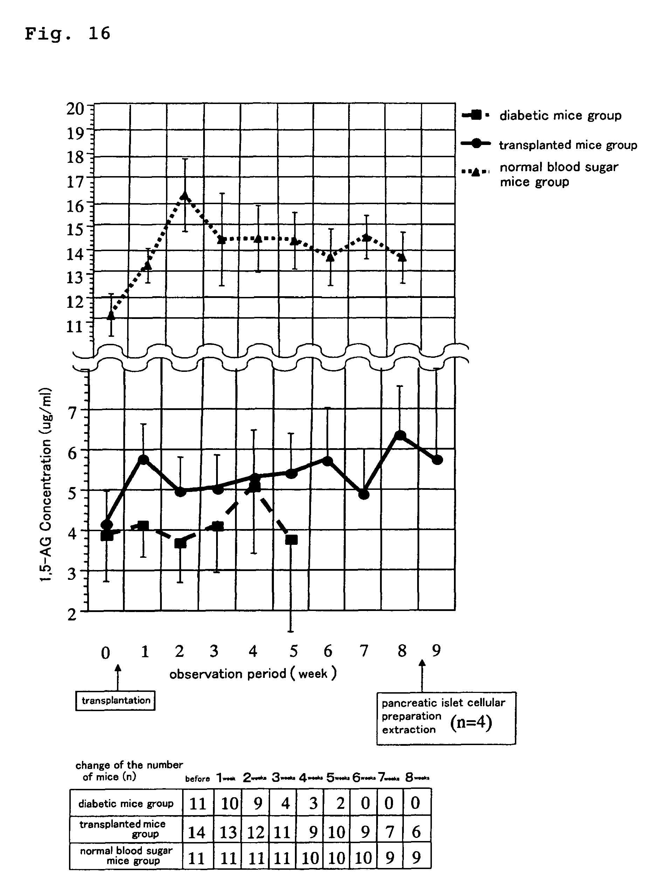

The results are shown in Fig. 16. As being made clear from the drawing, as compared with the group of the normal blood sugar mice, the group of diabetic mice showed apparently low 1.5-AG concentration value, well reflecting the diabetic state. The average value of the group of the transplanted mice was increased within 1 week as compared with the value before the transplantation. As compared with the value of the group of the diabetic mice, significant increase was observed (p = 0.0203).

### (8) Conclusion

As clearly shown from the above described transplantation experiment, the pancreatic islet cellular preparation of the present invention is effective for improving the diabetic state and preventing the death from diabetes and diabetic renal insufficiency by transplantation of the preparation. The transplantation of the pancreatic islet cellular preparation of the present invention is not only effective in treatment for diabetes but also in prevention of diabetic complication.

### INDUSTRIAL APPLICABILITY

The present invention can provide a cellular preparation capable of stably and durably retaining cells having capabilities of producing/secreting biologically active factors such as hormones and proteins useful for patients in PVA, which is a proper material as a high molecular weight polymer to be used for bioartificial organs. Further, by administration of the cellular preparation to patients, endocrine/ metabolic disease, hemophilia, systemic bone disease or cancer may be prevented or treated. Since the pancreatic cellular preparation of the present invention can retain cells stably for a long duration, resulting in lessened frequency of transplantation and may be formed in various shapes, it is possible to transplant such cellular preparation by subcutaneous or intramuscular injection.

## Claims

1. A cellular preparation comprising cells in polyvinyl alcohol admixed with a cell preservative.

2. The cellular preparation as claimed in claim 1, wherein the cell surface is further coated with an extracellular matrix.

3. The cellular preparation as claimed in claim 1 or 2 further comprising a growth factor.

4. The cellular preparation as claimed in any one of claims 1 to 3, wherein the cells are one or two or more kinds of cells selected from the group consisting of pancreatic islet cells, pancreatic endocrine cells, hepatocytes, anterior pituitary cells, growth hormone-secreting cells, osteocytes, thyroid hormone-secreting cells, and parathyroid hormone-secreting cells.

5. The cellular preparation as claimed in any one of claims 1 to 4, wherein the cells are treated with a cell preservative.

6. The cellular preparation as claimed in any one of claims 1 to 5, wherein the cells are transformed cells.

7. The cellular preparation as claimed in any one of claims 1 to 6, which has a sheet-like, plate-like, rod-like, tubular, or bead like shape.

8. The cellular preparation as claimed in any one of claims 1 to 7, wherein the cell preservative solution is Euro-Collins solution, Cell Banker, or UW solution.

9. The cellular preparation as claimed in any one of claims 1 to 8, which is transplanted subcutaneously, intramuscularly, or intraabdominally to mammals.

10. A method for prevention and treatment of endocrine or metabolic disease, which comprises using the cellular preparation according to any one of claims 1 to 9.

11. The method for prevention and treatment of endocrine or metabolic disease as claimed in claim 10, wherein the endocrine or metabolic disease is diabetes or pituitary dwarfism.

12. A method for prevention and treatment of hemophilia, which comprises using the cellular preparation according to any one of claims 1 to 9.

13. A method for prevention and treatment of bone disease, which comprises using the cellular preparation according to any one of claims 1 to 9.

14. A method for prevention and treatment of cancer, which comprises using the cellular preparation according to any one of claims 1 to 9.

15. A method for prevention and treatment of hepatic insufficiency or inborn errors of metabolism, which comprises using the cellular preparation according to any one of claims 1 to 9.

16. A method for preparing a cellular preparation, which comprises admixing a cell preservative with a polyvinyl alcohol, and mixing cells with the polyvinyl alcohol, and gelling the resulting cell-containing polyvinyl alcohol.

17. The cellular preparation as claimed in any one of claims 1 to 9, which is a medicine for human or animal use.
